# EUROPÄISCHE PATENTSCHRIFT

(11) **EP 0 396 615 B1**
(45) Veröffentlichungstag und Bekanntmachung des Hinweises auf die Patenterteilung: **21.04.1993**
(21) Anmeldenummer: 89901842.8
(22) Anmeldetag: 02.02.1989
(51) Int. Cl.: A61B 6/04, H05B 3/34, A61G 13/00

(54) **WÄRMESYSTEM FÜR EINEN OPERATIONSTISCH**
HEATING SYSTEM FOR AN OPERATING TABLE
SYSTEME DE CHAUFFAGE POUR TABLES D'OPERATION

(30) Priorität: 03.02.1988 DE 3803139
(43) Veröffentlichungstag der Anmeldung: 14.11.1990
(73) Patentinhaber: STIHLER ELECTRONIC MEDIZINTECHNISCHE GERÄTE PRODUKTIONS- UND VERTRIEBS-GMBH, D-70597 Stuttgart (DE); Horn GmbH + Co. KG, 78244 Gottmadingen (DE)
(72) Erfinder: THEILACKER, Wolfgang, D-7000 Stuttgart 70 (DE); STIHLER, Hans-Peter, D-7000 Stuttgart 70 (DE); STIHLER, Axel, D-7000 Stuttgart 70 (DE); BURKERT, Josef, D-7763 Öhningen-Wangen (DE)
(74) Vertreter: KOHLER SCHMID + PARTNER
(86) Internationale Anmeldenummer: DE8900065
(87) Internationale Veröffentlichungsnummer: WO8906931

(56) Entgegenhaltungen:
- BE-A- 688 786
- DE-A- 2 157 356
- FR-A- 1 390 568
- FR-A- 2 341 287
- FR-A- 2 355 426

## Beschreibung

Die Erfindung geht aus von einem Wärmesystem für einen Operationstisch mit einer mehrschichtig aufgebauten Heizauflage, die mindestens ein elektrisch beheizbares, für Röntgenstrahlen durchlässiges Heizsegment aufweist, das an ein Regel- und Steuergerät angeschlossen ist, das eine Regelung oder Steuerung eines für eine einstellbare Temperatur erforderlichen Heizstromes vermittelt und die aus unterschiedlichen Schichten gebildet ist, die untereinander einen innigen Materialverbund bilden.

Bekannte Heizauflagen für OP-Tische sind verhältnismäßig dick, entweder weil ihr Heizleiter aus einem leitfähig gemachten Gummi- oder Kunststoff besteht, der eine gewisse Plattendicke nicht unterschreiten kann, damit diese Platte eine hinreichend große Stromstärke aufnimmt, die für eine angemessene Heizleistung erforderlich ist und/oder weil Polsterschichten, die die eigentliche Heizauflage umgeben, eine entsprechende Dicke aufweisen. Dicke bzw. gepolsterte Heizauflagen lassen sich jedoch schlecht zusammenlegen, ihr Wärmedurchgang verschlechtert sich mit zunehmender Dicke und sie lassen sich beispielsweise auch schlecht in Gefäße einlegen, in denen diese Heizauflagen sterilisiert werden sollen. Gegebenenfalls sind bekannte Heizauflagen für eine Sterilisation schon deshalb weitgehend ungeeignet, weil sie aus einzelnen unverbundenen Schichten aufgebaut sind und die verwendeten Bauelemente durch eine Sterilisation beschädigt werden würden.

Eine derartige Heizauflage für OP-Tische ist durch den Prospekt VITALMED Thermoauflage VIT 2000 oder aus der BE-A-688 786 bekannt geworden.

Die bekannten Heizauflagen haben jedoch den Nachteil, daß die flächenartigen Heizleiter nur für eine begrenzte Heizleistung geeignet sind und bei einer weiteren Heizleistungssteigerung unzumutbar dick und unbeweglich werden würden. Die bekannten Heizauflagen sind schon in der bestehenden Ausführungsform so dick, daß sie sich nicht zusammenlegen bzw. -rollen lassen. Ferner weisen sie Bauteile auf, die bei einem Zusammenrollen den Schichtaufbau der Heizauflagen beschädigen können. Die Bauteile sind nicht so weit temperaturbeständig, daß die Heizauflagen sterilisiert werden können.

Ferner werden bei der Heizauflage Vitalmed Thermoauflage VIT 2000 die jeweils innerhalb eines Heizfeldes ermittelten Temperaturen dieses Heizfeldes in der Heizauflage selbst verglichen und an das Regel- und Steuergerät wird jeweils nur ein Temperatur-Ist-Wert pro Heizfeld übermittelt.

Eine weitere Heizauflage ist unter der Warenbezeichnung "Thermomaquet 1004.64" bekanntgeworden.

Diese Heizauflage ist nicht in getrennt betriebene Heizfelder unterteilt und weist zudem eine sich nahezu über die gesamte Fläche dieser Heizauflage erstreckende, flächenhaft verlegte Aluminiumfolie auf, die Dehnungen der einzelnen Schichten der Heizauflage entgegenwirkt. Deshalb kann diese Heizauflage auch schlecht zusammengelegt oder -gerollt werden und ist für eine Sterilisation ungeeignet.

Weiterhin ist aus der DE-OS 31 19 757 eine Regeleinrichtung zur Regelung der Oberflächentemperatur eines beheizbaren Polsters, insbesondere des Polsters eines Operationstisches, bekanntgeworden, bei dem der Fühler gegenüber dem Polster beweglich und flach ausgebildet ist. Der Temperaturfühler ist im Polster von steifen Kupferplatten umgeben.

Damit hat das bekannte Heizpolster den Nachteil, daß eine überwachung der Heizleistung aus sicherheitstechnischen Gesichtspunkten nur begrenzt möglich ist. Ein weiterer Nachteil ist, daß die mit Kupferplatten umgebenen Temperaturfühler nicht röntgenstrahlendurchlässig sind und auf mittels Röntgenstrahlen hergestellten Bildern unerwünschte Kontraste erzeugen. Deshalb müssen derartige Temperaturfühler im Randbereich eines Heizpolsters angeordnet werden und können deshalb Temperatur-Ist-Werte in der Heizzone nicht erfassen. Zudem ist der plattenartige Aufbau der Temperaturfühler und der mehrschichtige Aufbau des Heizpolsters selbst nicht dafür geeignet, daß man das Heizpolster für Sterilisationszwecke eng zusammenrollt.

Aus dar DE-PS 715 880 ist ein Heizleiter für ein schmiegsames, elektrisches Wärmegerät bekanntgeworden, das einen Heizleiter aufweist, dessen Tragfaden aus Asbest oder einem sonstigen Isolierstoff bestehen kann. Der Tragfaden ist mit einem oder mehreren blanken oder isolierten Heizdrähten in schraubenförmigen Windungen umgeben. Der bekannte Heizleiter hat den Nachteil, daß sein Tragfaden nicht dehnbar ist und deshalb dieser Tragfaden mit dem darauf gewickelten Widerstandsdraht schon bei den geringsten Belastungen beschädigt werden kann. Da erfahrungsgemäß Heizauflagen für OP-Tische gut biegbar und dünn sein sollen, damit sie sich an unterschiedliche Stellungen des OP-Tisches ohne aufzutragen anpassen ist der bekannte Heizleiter für Heizauflagen im OP-Bereich ungeeignet.

Schließlich ist aus der DD-PS 216 627 eine Wärmematte für medizinische Anwendungen bekannt geworden, die flexibel und sterilisierbar sein soll und ebenfalls den Anforderungen der technischen Sicherheit in der Medizin entsprechen soll. Dabei ist die Heizauflage, wie die Fig. 2 dieser Druckschrift zeigt, aus mehreren einzelnen Schichten im losen Verbund zueinander zusammengesetzt, die von einer Hülle aus dünner Kunstlederfolie zusammengehalten werden. Als Heizleiter wird eine Metallgaze verwendet. Der beschriebene Schichtaufbau ist weder dampfdicht noch sind die einzelnen Einbauten der Heizauflage so ausgeführt oder geschützt, daß die Heizauflage als ganzes zusammengerollt oder sterilisiert werden könnte. Handelsübliche Metallgazen sind nicht biegestabil und sind auch nicht für Röntgenstrahlen durchlässig. Weiterhin kann mittels der beschriebenen Metallgaze keine große spezifische Heizleistung erreicht werden.

Der Erfindung liegt daher die Aufgabe zugrunde, eine Heizauflage der eingangs genannten Art dahingehend weiterzubilden, daß sie auch bei erhöhter Heizleistung möglichst dünn gefertigt werden kann und daß die Heizauflage einen Aufbau aufweist, der Einbauten bestmöglichst schützt und der eine sichere Überwachung der Heizleistung zuläßt.

Diese Aufgabe wird erfindungsgemäß dadurch gelöst, daß die Heizauflage einen Heizleiter aufweist, der einen mit einem bestimmten elektrischen Widerstand behafteten Leiter umfaßt, der auf einen Träger schraubenförmig aufgewickelt ist, der aus einem elektrisch nicht leitenden Material besteht, das hinsichtlich seiner mechanischen Eigenschaften in einem Temperaturbereich von T = 10° Celsius bis T = 200° Celsius gummielastisch ist.

Die erfindungsgemäße Heizauflage hat damit den wesentlichen Vorteil, daß sie besonders dünn gefertigt werden kann, ohne daß dabei die Heizleistung der Heizauflage eingeschränkt werden müßte. Der Heizleiter kann zum Beispiel nur einen Durchmesser von 1,3 mm aufweisen. Durch die Ausgestaltung des Heizleiters in der Form, daß der Leiter spiralförmig auf den Träger aufgewickelt ist und daß der Träger gummielastisch ist, kann sich der Leiter in Achsrichtung auch dehnen. Die Dehnbarkeit des Heizleiters wird nur durch die Dehnfähigkeit des Trägermaterials begrenzt. Ferner verjüngt sich der Träger bei einer axialen Dehnung und dabei wirkende Kräfte werden nicht auf den Leiter übertragen.

Der Heizleiter ist in hohem Maße biegsam, so daß er sich nicht nur Verformungen der Heizauflage anpaßt, sondern es müssen auch keine besonderen Maßnahmen im Schichtaufbau der erfindungsgemäßen Heizauflage zum Schutz des Heizleiters erfolgen. Zusätzliche Schichten oder Umhüllungen zum Schutz des Heizleiters können entfallen. Dadurch wird der dünne Aufbau der erfindungsgemäßen Heizauflage begünstigt. Weiterhin kann der Heizleiter aufgrund seiner guten Biegsamkeit derart in der Heizauflage verlegt werden, daß auch eine optimale Überwachung der Heizleistung durch geeignete Bauteile möglich ist.

Durch eine Vernetzung der aneinandergrenzenden Schichtflächen besteht ein dichter Kontakt der Schichten untereinander, der auch dann bestehen bleibt, wenn die Heizauflage verformt wird. Dies ist eine Voraussetzung für einen guten Wärmeübergang vom Heizleiter auf angrenzende Schichten. Dadurch, daß auch eine Materialvernetzung zwischen dem Träger des Heizleiters und der den Heizleiter umgebenden Schicht eintritt, kann der Heizleiter sehr eng zum Beispiel mäanderförmig in der Heizauflage geführt werden. Der Heizleiter ist in der Heizauflage fest fixiert, so daß gegenseitige Berührungen der Heizleiter untereinander ausgeschlossen werden können. Diese konstruktive Ausgestaltung der erfindungsgemäßen Heizauflage gewährleistet erstens eine Heizleistungssteigerung auf engem Raum, zweitens einen guten Schutz des Heizleiters und drittens kann die Heizleistung einzelner Heizleiterabschnitte sicher überwacht werden.

Die erfindungsgemäße Heizauflage ist durch den Materialverbund der Schichten grundsätzlich dampfdicht, d.h., sie kann mit gespanntem Sattdampf den medizinischen Vorschriften für die Sterilisation von Bestecken u.s.w. entsprechend sicher sterilisiert werden, ohne daß Bauteile im Innern der Heizauflage beschädigt werden.

Ist die Gesamtdicke der Heizauflage im Heizsegmentbereich ca. 5 mm und im Randbereich ca. 8 mm, so bleibt die erfindungsgemäße Heizaflage eng zusammenrollbar und flexibel. Die Herstellung einer derart dünnen Heizauflage mit einer zufriedenstellenden Heizleistung ist nur in Kombination der beschriebenen Merkmale möglich.

Weiterhin ist der Heizleiter in eine erste Schicht eingebettet, die eine Dicke aufweist, die dem Außendurchmesser des Heizleiters entspricht, oder bevorzugt 10 % bis 20 % dicker ist als der Außendurchmesser des Heizleiters.

Dies hat den Vorteil, daß bei der Vulkanisation eine gute und stabile flächenhafte Vernetzung mit einer an die erste Schicht angrenzenden Schicht erfolgt.

Die erste Schicht ist von einer zweiten Schicht aus Siliconkautschuk umgeben, die erste und die zweite Schicht weisen weitgehend dieselben Materialeigenschaften auf und die elastische Ausdehnung der ersten und der zweiten Schicht ist durch eine zwischen der ersten und der zweiten Schicht angeordneten Gewebeschicht begrenzt, die mit den angrenzenden Schichten einen Materialverbund bildet.

Dies hat den Vorteil, daß sich ein Materialverbund zwischen der ersten und der zweiten Schicht auch dann einfach durch eine Vulkanisation herstellen läßt, wenn zum Beispiel für die erste und zweite Schicht unterschiedliche Siliconkautschukmaterialien ausgewählt werden. Dies deshalb, weil die Eigenschaften der Materialien bezüglich ihrer Verarbeitung nahezu gleich bleiben. Das Siliconkautschukmaterial für die zweite Schicht kann zum Beispiel so ausgewählt werden, daß es weicher und im Oberflächenbereich widerstandsfähiger ist als das Siliconkautschukmaterial der ersten Schicht. Dadurch ergibt sich ein verbesserter Liegekomfort und die Heizauflage ist bezüglich der Reinigung und mechanischer Einflüsse widerstandsfähiger.

Durch die Gewebeschicht können die gummielastischen Eigenschaften der Siliconkautschukschichten sinnvoll eingegrenzt werden. Ferner wird die Formstabilität der Heizauflage erhöht. Wählt man als Gewebeschicht, z.B. eine Glasfasermatte aus, so ergibt sich bei der Vulkanisation der Heizauflage auch ein Materialverbund zwischen den Siliconkautschukschichten und der Gewebeschicht.

In weiterer Ausgestaltung der Erfindung weist die Heizauflage einen Heizleiter auf, der in der biegetechnisch neutralen Mittelebene der Heizauflage verlegt ist.

Dies hat den Vorteil, daß der Heizleiter durch auf die Heizauflage wirkende Längs- und/oder Querkräfte nicht beschädigt werden kann. Auch ein kleiner Radius beim Zusammenrollen der Heizauflage kann den Heizleiter nicht beschädigen.

In bevorzugter Ausgestaltung der Erfindung setzt sich der Heizleiter aus einem Träger, bevorzugt aus Siliconkautschuk, und aus einem im Querschnitt kreisförmigen Aluminiumdraht als Leiter zusammen.

Der Träger aus Siliconkautschuk ist im Temperaturarbeitsbereich der Heizauflage in hohem Maße gummielastisch und bewirkt zusammen mit dem im Querschnitt kreisförmigen Leiter und der spiralförmigen Führung des Leiters auf dem Träger, daß der ansonsten mechanisch nicht stark belastbare Leiter aus Aluminium elastische Eigenschaften bekommt. Ein derart erfindungsgemäß aufgebauter Heizleiter schafft erst die Voraussetzungen, daß eine Heizauflage für OP-Tische dünn, mit hoher Heizleistung, röntgenstrahlendurchlässig und einem sicher zu überwachenden Heizsystem hergestellt werden kann.

In weiterer Ausgestaltung der Erfindung ist die erste Schicht eine elektrisch nicht leitende Siliconkautschukschicht, die eine gute Wärmeleitfähigkeit aufweist und im Materialverbund ein dem Träger ähnliches gummielastisches Verhalten aufweist. Die Ausbildung der ersten Schicht aus Siliconkautschuk hat den Vorteil, daß sie eine ähnliche Dichte wie das Trägermaterial aufweist und sich auf diese Art und Weise einfach ein Materialverbund zwischen dem Träger des Heizleiters und der ersten Schicht herstellen läßt. Durch die gleichen gummielastischen Eigenschaften sind beim Dehnen der Heizauflage Materialverwerfungen ausgeschlossen.

In weiterer Ausgestaltung der Erfindung ist die zweite Schicht eine elektrisch nicht leitende Siliconkautschukschicht, die im Randbereich verstärkt ist und die über die Oberfläche mit einer weiteren dritten Schicht im Verbund steht, die elektrische Ladungen ableiten kann. Durch die Verstärkung der zweiten Schicht im Randbereich wird die Formstabilität der Heizauflage weiter erhöht, die Verformbarkeit der Heizauflage aber nicht eingeschränkt. Die Heizauflage erhält eine rahmenhafte Führung. Die elektrisch nicht leitende zweite Schicht steht ferner im Materialverbund mit einer dritten Schicht, die elektrisch leitfähige Eigenschaften aufweist. Über die dritte Schicht lassen sich elektrische Ladungen ableiten.

Weiterhin sind in der Heizauflage zwischen einem mäanderförmig geführten Heizleiter Temperaturfühler, bevorzugt zwei Halbleiterwiderstände mit großen negativen Temperaturkoeffizienten je Heizsegment, angeordnet.

Dies hat den Vorteil, daß der Heizleiter nicht nur nicht verschiebbar in der dünnen ersten Schicht sicher geführt ist und durch die enge Ummantelung des Heizleiters ein guter Wärmeaustausch zwischen der ersten Schicht und dem Heizleiter gegeben ist, sondern daß auch ebenfalls die Temperaturfühler (NTC) durch das Einvulkanisieren in die erste Schicht in derselben Ebene wie der Heizleiter sicher, exakt und unverrückbar positioniert sind. Die NTC-Temperaturfühler schränken auch nicht die Biegsamkeit der ersten Schicht ein, weil sie bauartbedingt jeweils nicht viel größer als ein Nadelkopf sind.

In weiterer Ausgestaltung der Erfindung sind die Temperaturfühler mit röntgenstrahlendurchlässigen Leitungen, die gummielastische Eigenschaften aufweisen, verdrahtet.

Dies hat den Vorteil, daß die Temperaturfühler an beliebigen Stellen in der Heizauflage der ersten Schicht sicher positioniert werden können. Die NTC-Temperaturfühler sind zwar nicht aus röntgenstrahlendurchlässigen Materialien aufgebaut, sie wirken aber auf einem Röntgenbild nicht als störend, weil sie sehr klein sind. Als störend für die Auswertung von Röntgenbildern würde man die Verschattungen von Ab- und Zuleitungen der NTC-Temperaturfühler empfinden, weil sich solche Verschattungen über die ganze Bildlänge bzw. Bildbreite erstrecken können. Wählt man als elektrische Zu- und Ableitungen für die NTC-Temperaturfühler einen elektrischen Leiter wie zum Beispiel den erfindungsgemäßen Heizleiter aus, so können die NTC-Temperaturfühler sicher in der Heizauflage verdrahtet werden und sind auf einem Röntgenbild nicht sichtbar. Die Leitungen sind biegsam und gummielastisch und können somit auch durch eine Verformung der Heizauflage nicht beschädigt werden.

In weiterer Ausgestaltung der Erfindung sind die elektrischen Leiter der Heizleiter der Heizsegmente in einer Parallelschaltung und die elektrischen Leiter der Temperaturfühler paarweise zusammengefaßt, und für die Ableitung elektrostatischer Aufladungen ist ein elektrischer Leiter vorgesehen, der mit einer dritten Schicht verbunden ist.

Über die beschriebene Verschaltung der Heizsegmente kann eine Störung schnell erkannt werden. Ferner muß nur eine elektrische Zu- und Ableitung in der Heizauflage geführt werden. Durch die Verdrahtung der NTC-Temperaturfühler ist eine sichere Temperaturmessung in der Heizauflage auch dann möglich, wenn die Heizauflage nur zu einem Drittel bedeckt ist.

Durch die elektrostatisch ableitfähige dritte Schicht sind elektrostatische Aufladungen oder Aufladungen durch hochfrequente Ströme im Bereich der Heizauflage ausgeschlossen, sie können sicher über diese dritte Schicht abgeleitet werden. Ebenfalls elektrisch leitend mit dieser dritten Schicht kann eine Abschirmung verbunden werden.

In weiterer Ausgestaltung der Erfindung weisen die elektrischen Leiter als Leiterbündel am Austritt aus der ersten Schicht einen daran anvulkanisierten Knickschutz auf, der bevorzugt mit einer Knickkerbe versehen ist.

Dies hat den Vorteil, daß bei einem Zusammenlegen bzw. -rollen der Heizauflage das Leiterbündel eng an die Heizauflage angelegt werden kann, ohne daß dabei die einzelnen Leiter im Leiterbündel abgeknickt werden würden.

In bevorzugter Ausgestaltung der Erfindung ist das Leiterbündel außerhalb der ersten Schicht mit einer im Bereich von T = 10° Celsius bis T = 200° Celsius temperaturbeständigen und biegsamen Ummantelung umgeben und das Leiterbündel mündet in einen Stecker, der wahlweise mit einer Kappe dicht verschlossen werden kann.

Dies hat den Vorteil, daß die Heizauflage gemeinsam mit dem anvulkanisierten Stromkabel und dem daran anschließenden Stecker eng zusammengerollt und sterilisiert werden kann. Mit der Kappe kann die offene Seite des Steckers druckdicht verschlossen werden, so daß beim Sterilisieren keine Feuchtigkeit in das Innere des Steckers gelangen kann.

Weiterhin sind bevorzugt auf der Unterseite der Heizauflage im Randbereich Verschlußbänder, bevorzugt Klettenbänder und/oder Druckknopfbänder, vorgesehen.

Dies hat den Vorteil, daß je nach Bedarf an der Heizauflage weitere Segmente einfach und sicher befestigt werden können.

In einer Weiterbildung der Erfindung ist die Heizauflage als Heizauflage mit einem Schlitz, der sich von einer Querseite der Heizauflage ausgehend teilweise in Längsrichtung erstreckt, ausgebildet, an das verschiedene Segmente anheftbar und/oder anknüpfbar sind.

Dies hat den Vorteil, daß die Segmente, wenn sie auf den Patienten oder um seine Extremitäten gelegt werden, nicht verrutschen können und den ganzen Patienten ohne freie Spalte abdecken. Das Wohlbefinden eines Patienten kann damit gefördert werden und einer zu starken Wärmeabstrahlung des Patienten über die Haut kann vorgebeugt werden.

Weiterhin weist das Regel- und Steuergerät mehrere Eingänge für Leitungen der Heizauflage und der Segmente auf.

Dies hat den Vorteil, daß komponentenartig verschiedene Heizauflageformen je nach Bedarf zusammengestellt werden können. Da sie über eigene Segmente und Zuleitungen verfügen, können sie auch einzeln betrieben werden, und sie sind getrennt von der Grundheizauflage sterilisierbar.

In weiterer Ausbildung der Erfindung sind die Heizauflage und/oder die Segmente mit einer Isolationsmatte abdeckbar.

Dies hat den Vorteil, daß die erfindungsgemäße Heizauflage auch dann verwendet werden kann, wenn Hochfrequenz-Chirurgiegeräte bei Operationen verwendet werden. Die elektrischen Geräte stören sich nicht gegenseitig und die sichere Funktion der hochfrequenzbetriebenen Chirurgiebestecke sowie der Heizauflage ist gewährleistet.

Bei einer weiteren Ausgestaltung der Erfindung weist das Regel- und Steuergerät ein Display auf, an dem ein erster und zweiter Schalter für die Temperaturvorwahl der Heizauflage und der Segmente und Leuchtfelder vorgesehen sind, die den Betrieb, den Betriebszustand und den Störfall der einzelnen Segmente anzeigen.

Dies hat den Vorteil, daß die Heizauflage einfach überwacht und besonders übersichtlich bedient werden kann.

Das erfindungsgemäße Wärmesystem für OP-Tische entspricht damit allen erweiterten Anforderungen, die im OP-Bereich gestellt werden. Die Heizauflage läßt sich leicht handhaben, trägt, weil sie besonders dünn ist, auf dem OP-Tisch nicht auf, die Heizauflage kann Verformungen in Längs- und Querrichtung aufnehmen, ohne daß dadurch Beschädigungen an der Heizauflage selbst entstehen oder eine Heizleistungsbeeinträchtigung erfolgen könnte, die Heizauflage ist in ihrer Größe beliebig erweiterbar, die einzelnen Heizauflagen bzw. -segmente können von einem Regel- und Steuergerät auch getrennt beheizt und temperaturgesteuert werden und jede einzelne Heizauflage bzw. jedes einzelne Segment ist besonders biegsam und damit eng zusammenrollbar und kann in handelsüblichen Sterilisationsgefäßen sterilisiert werden. Die Heizauflage ist schwer entflammbar.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen in der Zeichnung näher erläutert. Es zeigen:
- Fig. 1: eine Zusammenstellung der einzelnen Komponenten des erfindungsgemäßen Wärmesystems für einen Operationstisch;
- Fig. 2: eine Heizauflage eines erfindungsgemäßen Wärmesystems in einer Schnittdarstellung II-II gemäß Fig.1;
- Fig. 3: ein Teilstück eines Heizleiters der Heizauflage;
- Fig. 4: eine Draufsicht auf die Heizauflage im Schnitt IV-IV gemäß Fig. 1;
- Fig. 5: einen Stecker mit Kappe, wie er am von der Heizauflage abweisenden Leitungsende bei der Sterilisation einer Heizauflage eingesetzt wird;
- Fig. 6: eine Draufsicht auf die Unterseite einer Heizauflage;
- Fig. 7: weitere Ausführungsbeispiele von einer Heizauflage und Segmenten in Draufsicht;
- Fig. 8: ein Ausführungsbeispiel eines Displays eines Steuerund Regelgerätes eines erfindungsgemäßen Wärmesystems für Operationstische.

Die einzelnen Figuren der Zeichnung zeigen teilweise stark schematisiert den erfindungsgemäßen Gegenstand und sind nicht maßstäblich zu verstehen. Die Gegenstände der einzelnen Figuren sind teilweise stark vergrößert dargestellt, damit ihr Aufbau besser gezeigt werden kann.

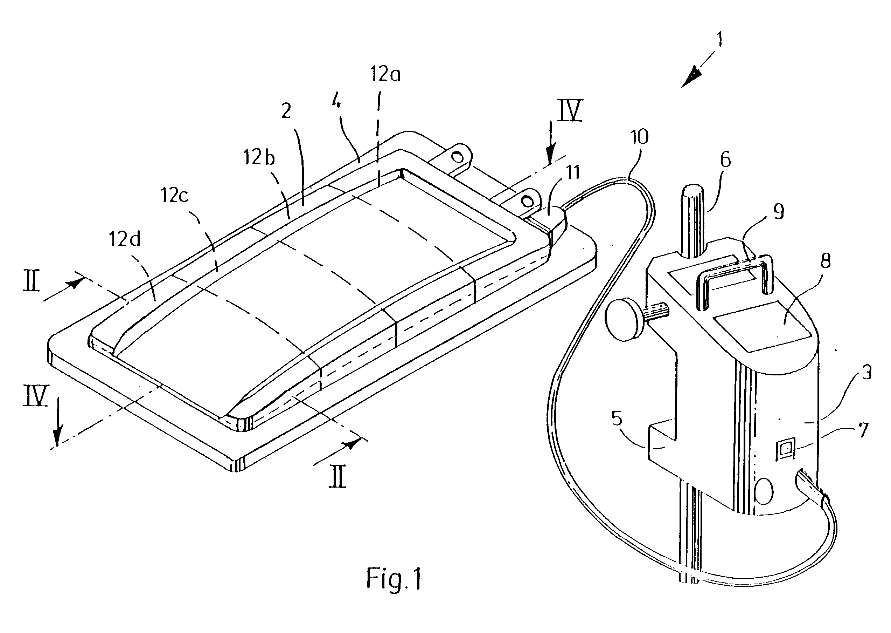

In Fig. 1 ist ein Wärmesystem für Operationstische 1 dargestellt, das sich aus einer Heizauflage 2 und einem Regel- und Steuergerät 3 zusammensetzt. Je nach Bedarf weist das Wärmesystem 1 noch eine Isolationsmatte 4 auf, die auf die Oberfläche der Heizauflage 2 und/oder unter die Heizauflage 2 gelegt werden kann. Der Einfachheit halber ist in der Fig. 1 nur eine Isolationsmatte 4 dargestellt. Die erfindungsgemäße Heizauflage 2 ist im Randbereich rahmenhaft verstärkt. Das Regel- und Steuergerät 3 weist eine Befestigungsklaue 5 auf, mit der es an einem Stab 6, zum Beispiel eines Infusionsständers, befestigbar ist. Das Gehäuse des Regel- und Steuergerätes 3 ist jedoch so ausgebildet, daß es auch als Standgerät aufgestellt werden kann. Auf der Frontseite des Regel- und Steuergerätes 3 ist ein Netzschalter 7 vorgesehen, mit dem das Wärmesystem 1 in Betrieb genommen werden kann. Die Frontfläche des Regel- und Steuergerätes 3 ist nach oben verlaufend nach hinten abgeschrägt, auf ihr ist ein Display 8 angeordnet. Am Deckel des Gehäuses ist ein Tragegriff 9 angeformt, mit dessen Hilfe das Regel- und Steuergerät 3 sicher getragen und für Befestigungszwecke sicher gehalten werden kann.

Ein Stromkabel 10 verbindet die Heizauflage 2 elektrisch mit dem elektrischen Steuergerät 3. Auf der Rückseite des Regel- und Steuergerätes 3 befindet sich ein nicht dargestellter Steckkontakt, über den die Heizauflage 2 und das Regel- und Steuergerät 3 an das Stromnetz angeschlossen werden können.

Das der Heizauflage 2 zugewandte Ende des Stromkabels 10 ist an die Heizauflage 2 anvulkanisiert. Die feste Verbindung zwischen der Heizauflage 2 und dem Stromkabel 10 ist mit einem Knickschutz ausgestattet, der beispielsweise mit einer Knickkerbe 11 versehen ist.

Die Heizauflage 2 selbst ist in vier Heizsegmente 12a, 12b, 12c, 12d aufgeteilt, die von dem Regel- und Steuergerät 3 beheizt und temperaturgesteuert werden.

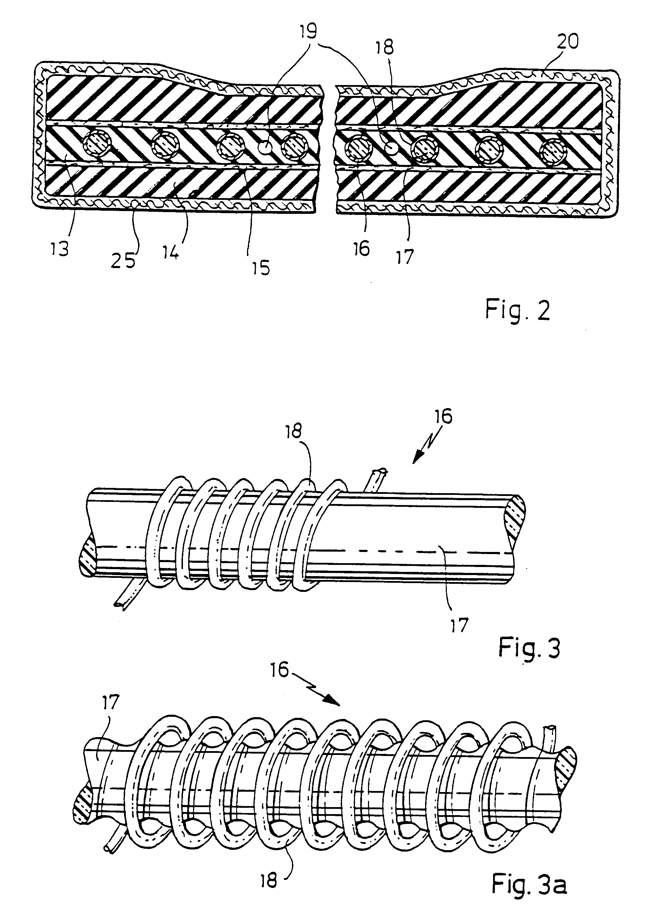

Fig. 2 zeigt den Schichtaufbau der Heizauflage 2 im Schnitt II-II der Fig. 1. Die Heizauflage 2 setzt sich aus einer elektrisch nicht leitenden ersten Schicht 13, einer zweiten Schicht 14, einer Gewebeschicht 15 und einer dritten Schicht 25 zusammen. Die erste Schicht 13, die zweite Schicht 14, die Gewebeschicht 15 und die dritte Schicht 25 bilden einen Materialverbund, der durch eine Vulkanisation der Schichten erreicht wird. Bei der ersten und zweiten Schicht 13, 14 handelt es sich um elektrisch nicht leitende Schichten. Die dritte Schicht 25 kann elektrostatische Aufladungen ableiten. Die Gewebeschicht 15 ist aus einem Material zu wählen, das bei der Vulkanisation der Schichten mit diesen einen Materialverbund eingehen kann.

Die Gewebeschicht 15, bevorzugt eine Matte aus Glasfasern, grenzt die gummielastischen Eigenschaften der aus Siliconkautschuk bestehenden ersten, zweiten Schicht 13, 14 ein.

Die erste Schicht 13 besteht aus einer Silicon-Kautschuk-Mischung, die im abvulkanisierten Zustand eine gute Wärmeleitfähigkeit aufweist und Bauteile eng und sicher ummanteln kann. Die zweite Schicht 14 besteht aus einer Silicon-Kautschuk-Mischung, die abriebfest ist und die die Weichheit der Heizauflage 2 bestimmt. Die dritte Schicht 25, auch eine Silicon-Kautschuk-Mischung, wird erst durch die Zumischung leitfähiger Bestandteile 20 in gewissem Umfang leitfähig. Leitfähige Bestandteile 20 können beispielsweise Ruß- oder Graphitpartikel sein. Durch die Anreicherung der dritten Schicht 25 mit leitfähigen Bestandteilen 20 ergibt sich für diese ein elektrischer Widerstand, der im Bereich zwischen 50.000 Ohm und 1 M Ohm liegt.

In die elektrisch nicht leitende erste Schicht 13 sind Heizleiter 16 einvulkanisiert, die aus einem Träger 17 und einem elektrischen Leiter 18 bestehen. Zwischen den Heizleitern 16 der ersten Schicht 13 sind Temperaturfühler 19 (NTC) einvulkanisiert, die die Temperatur der Heizleiter 16 erfassen und als Ist-Wert an das Regel- und Steuergerät 3 weiterleiten.

In der Fig. 2 sind die einzelnen Schichten nicht maßstabsgerecht dargestellt. Die Schichtdicke der dritten Schicht 25, die die Heizauflage 2 nicht nur auf den Längsseiten, sondern auch auf den Querseiten ummantelt und keinerlei Auswirkungen auf die mechanischen und chemischen Eigenschaften der Heizauflage 2 hat, beträgt nur wenige µ-Meter. Die Schichtdicke der ersten und zweiten Schicht 13, 14, sowie der Gewebeschicht 15 liegen im Milimeterbereich. Die Gesamtdicke der Heizauflage 2 beträgt im Heizsegmentbereich ca. 5 mm und im Randbereich ca. 8 mm.

Fig. 3 zeigt stark vergrößert ein Teilstück des Heizleiters 16, wie er in die erste Schicht 13 einvulkanisiert ist. Als Träger 17 wird ein gummielastisches elektrisch nicht leitendes Material verwendet, das in der eingesetzten Form im Querschnitt kreisförmig ist und einen Durchmesser von ca. 1,2 mm aufweist. Es können aber auch Träger eingesetzt werden, die einen Durchmesser von 1 mm bis zu 1,5 mm aufweisen. Als Träger 17 wird in der beschriebenen Ausführungsform ein in einem weiten Temperaturbereich gummielastisches Siliconkautschukmaterial eingesetzt.

Um den Träger 17 ist der elektrische Leiter 18 eng anliegend gewickelt, der als Aluminiumdraht oder als Aluminiumband, je nach Ausführungsform des Heizleiters 16, eine Dicke von ca. 0,2 mm bis 0,5 mm aufweist. Der Träger 17 verleiht dem dünnen, im Querschnitt kreisförmigen Aluminiumdraht die Festigkeit und die spiralförmige Aufwicklung des Leiters 18 auf den gummielastischen Träger 17 ermöglicht Dehnungen des Leiters 18 in Achsrichtung und Belastungen in Querrichtung, ohne daß diese den Heizleiter 16 beschädigen.

Fig. 3a zeigt stark vergrößert wie der Leiter 18 den Träger 17 umschlingt. In der Figur ist der Leiter 18 nur aus Zwecken der Darstellung nicht enganliegend am Träger 17 dargestellt. Die einzelnen Leiterabschnitte des Leiters 18 umgreifen den Träger 17 formschlüssig.

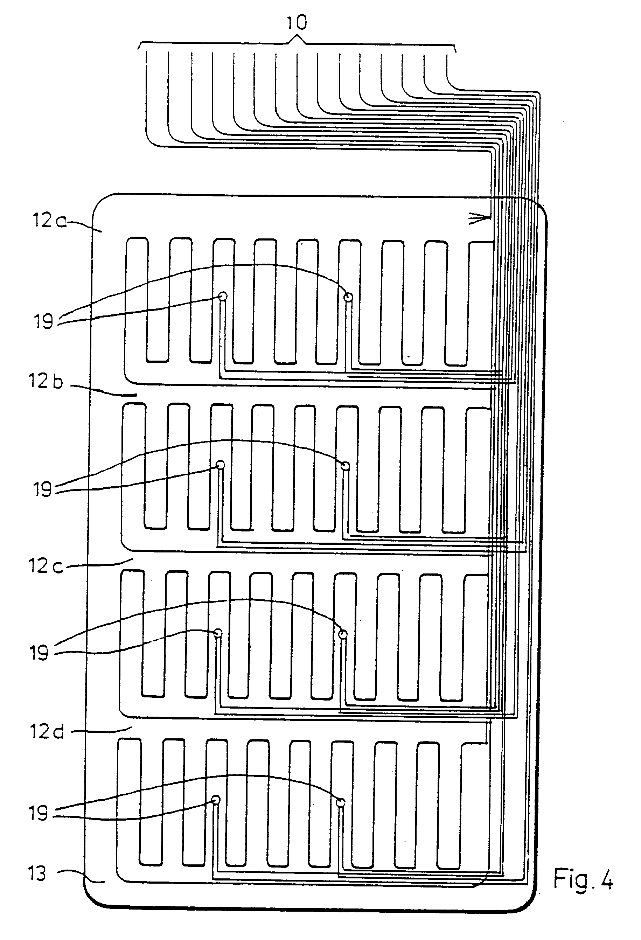

Fig. 4 zeigt einen Schnitt IV-IV der Heizauflage 2 gemäß Fig. 1. Die Draufsicht zeigt, wie die einzelnen Heizsegmente 12a, 12b, 12c, 12d in der ersten Schicht 13 angeordnet sind, die Positionierung der Temperaturfühler 19, die Führung der Heizleiter 16 in den einzelnen Heizsegmenten 12a, 12b, 12c, 12d und die Leiterführung bzw. Verdrahtung der Heizleiter 16 und der Leitungen der Temperaturfühler 19. Die elektrischen Leiter werden beispielhaft an der rechten Kopfseite aus der ersten Schicht 13 herausgeführt und dort zu einem Leiterbündel zusammengefaßt, das das Stromkabel 10 bildet. Die elektrischen Leiter werden am Austritt aus der ersten Schicht 13 nochmals an die Heizauflage 2 anvulkanisiert und zwar derart, daß kein Zug auf die elektrischen Leiter der Heizleiter 16 bzw. elektrischen Leiter der Temperaturfühler 19 in der ersten Schicht 13 übertragen wird, wenn zum Beispiel das Stromkabel 10 geknickt oder an ihm gezogen wird.

In der beispielhaften Ausführung der Fig. 4 sind die Heizsegmente 12a, 12b, 12c, 12d in einer Parallelschaltung elektrisch miteinander verbunden. Die elektrischen Leiter der Temperaturfühler 19 sind paarweise zusammengefaßt und werden so aus der ersten Schicht 13 herausgeführt. Ein weiterer Leiter ist für die Abschirmung des Stromkabels 10 und der Heizauflage 2 vorgesehen. Der elektrische Leiter für die Abschirmung ist mit der dritten Schicht 25 elektrisch verbunden.

Durch die Art der Verdrahtung der Heizsegmente 12a, 12b, 12c, 12d und der Temperaturfühler 19 wird ein hohes Maß an Sicherheit erreicht und das 15-adrige Leiterbündel, das Stromkabel 10, auf eine überschaubare Anzahl von Einzeldrahtleitern beschränkt.

Die Temperaturfühler 19 sind derart in den einzelnen Heizsegmenten 12a, 12b, 12c, 12d positioniert, daß sie in derselben Ebene wie der Heizleiter 16 liegen und sind mit elektrischen Leitern verbunden, die im Ausführungsbeispiel dem Heizleiter 16 entsprechen und für Röntgenstrahlen durchlässig sind.

Der mäanderförmige Verlauf des Heizleiters 16 in der Fig. 4 ist beispielhaft zu verstehen und kann bei Bedarf auch anders verlaufend in der ersten Schicht 13 verlegt werden.

Durch die Knickkerbe 11 am Austritt des Stromkabels 10 aus der Heizauflage 2 kann das Stromkabel 10 in einem weiten Bereich zug-und druckfrei um die Heizauflage 2 gebogen werden.

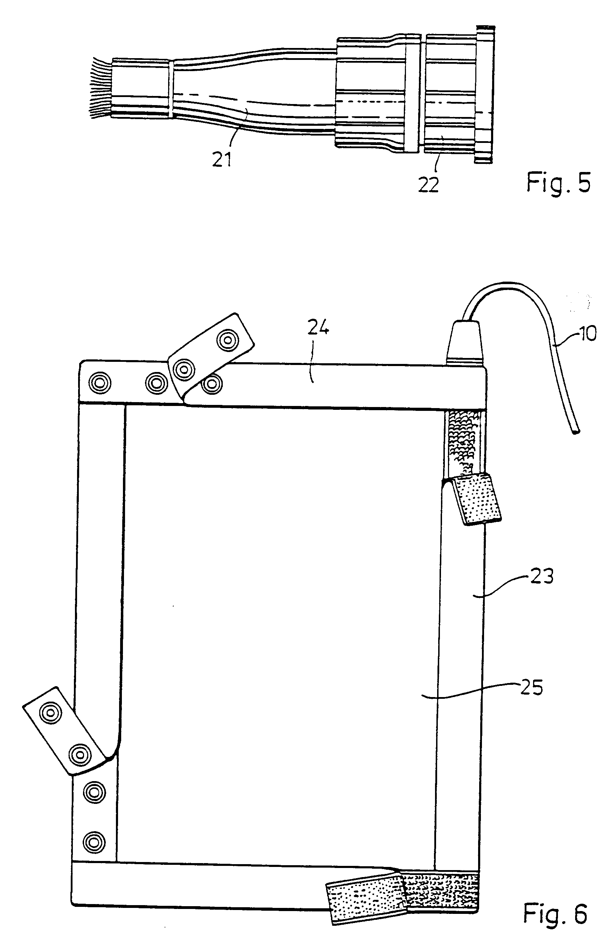

Fig. 5 zeigt einen Stecker 21, wie er am von der Heizauflage 2 abgewandten Ende des Stromkabels 10 vorgesehen ist. Auf das freie Ende des Steckers 21 ist eine Kappe 22 druck-, gas- und flüssigkeitsdicht aufsteckbar. Die Kappe 22 wird dann auf den Stecker 21 aufgesetzt, wenn die Heizauflage 2 zusammen mit dem daran angeformten Stromkabel 10 und dem Stecker 21 sterilisiert werden soll. Wird die Kappe 22 vom Stecker 21 abgenommen, so kann der Stecker 21 in eine auf der Rückseite des Regel- und Steuergerätes 3 vorgesehene Steckverbindung gesteckt werden.

Fig. 6 zeigt in einer Draufsicht die Unterseite der Heizauflage 2, d.h. die Oberfläche der leitfähigen dritten Schicht 25. Wie beispielhaft in der Figur gezeigt, sind im Randbereich auf der leitfähigen dritten Schicht 25 Verschlußbänder angebracht, die entweder mit dieser verklebt oder aufvulkanisiert sind. Bei den Verschlußbändern handelt es sich bevorzugt um Klettenbänder 23 oder Druckknopfbänder 24, über die weitere Heizauflagen an die Heizauflage 2 anheftbar sind. Damit kann die Heizauflage 2 auf einfache Weise vergrößert werden.

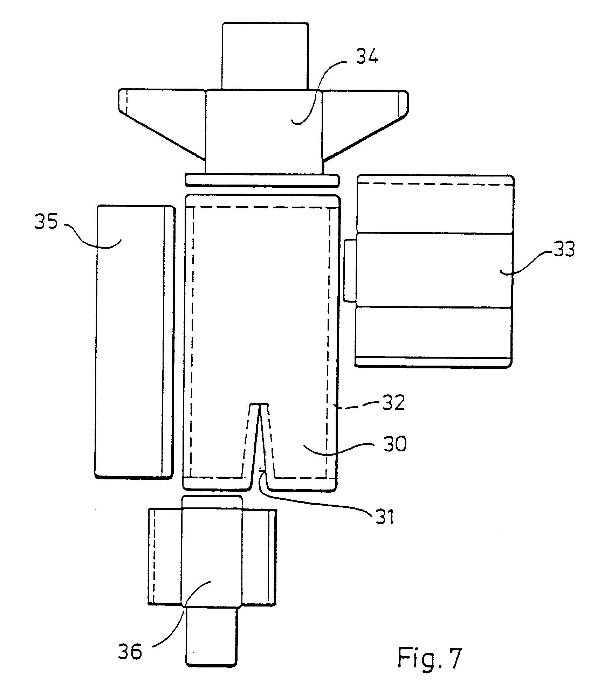

Fig. 7 zeigt stark schematisch eine weitere Ausführungsform einer Heizauflage 30 und weitere Ausführungsformen von Segmenten 33 bis 36.

Die Heizauflage 30 weist auf ihrer Unterseite im Randbereich Verschlußbänder 32 auf, über die die Segmente 33, 34, 35 und 36 an die Heizauflage 30 anknöpfbar bzw. anheftbar sind. Die Heizauflage 30 ist auf einer Querseite mittig mit einem Schlitz 31 versehen. Das Segment 33 ist beispielsweise so ausgebildet, daß es einerseits an die Heizauflage 30 anheftbar ist und andererseits um die Arme eines Patienten gelegt werden kann. Das Segment 34 ist so ausgeformt, daß es rutschfest um den Kopf eines Patienten gelegt werden kann. Das Segment 35 ist rechteckförmig als Decke ausgeformt, mit der ein Patient zugedeckt werden kann. Im Bein- und Fußbereich eines Patienten kann beispielsweise das Segment 36 so ausgeformt sein, daß es enganliegend um den Fuß und das Bein gelegt werden kann.

Die Gestaltungsformen der Segmente 33 bis 36 sind beispielhaft zu verstehen und beschränken sich nicht auf die dargestellten Ausführungsformen.

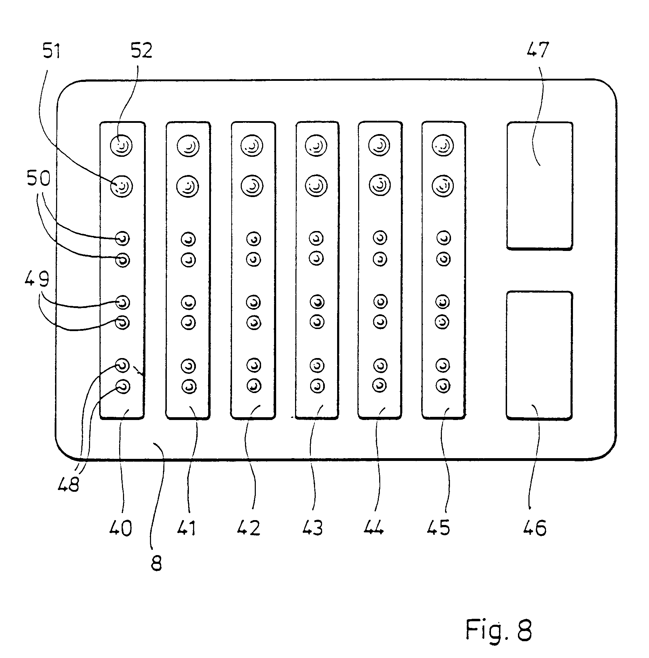

Fig. 8 zeigt beispielhaft eine Ausführungsform eines Displays 8 des Regel- und Steuergerätes 3. Auf dem Display 8 sind Leuchtfelder 40 bis 45 angeordnet, die jeweils einem Heizsegment 12a, 12b, 12c, 12d zugeordnet sind. So kann beispielsweise das Leuchtfeld 40 dem Heizsegment 12a, das Leuchtfeld 41 dem Heizsegment 12b, das Leuchtfeld 42 dem Heizsegment 12c und das Leuchtfeld 43 dem Heizsegment 12d zugeordnet sein. Die Leuchtfelder 44 und 45 sind freibelegbar und können Heizsegmenten von weiteren Heizauflagen 2/30 bzw. Segmenten 33 bis 36 zugeordnet werden. Das Display 8 weist ferner einen ersten Schalter 46 und zweiten Schalter 47 auf. Mit den Schaltern 46, 47 kann die Temperatur vorgewählt werden, mit der das Heizpolster 2, 30 bzw. die Heizpolstersegmente 33 bis 36 beheizt werden sollen. Die Schaltflächen der Schalter 46, 47 können für die optische Anzeige von Lagepositionen eines Patienten geteilt sein (z.B. Seitenlage), d.h. ist einer der Schalter 46, 47 gedrückt, so erscheint in einem Teil seines Druckfeldes ein optisches Signal, das anzeigt, ob die Heizauflage 2 so beheizt wird, daß sie beispielsweise entweder genug Wärme für die Seiten- oder Rückenlage eines auf der Heizauflage 2 liegenden Patienten abstrahlt.

Die Funktionsweise des in Fig. 1 dargestellten Wärmesystems für Operationstische ist wie folgt:
Um das Wärmesystem 1 in Betrieb zu nehmen, wird zuerst die Heizauflage 2 über das Stromkabel 10 mit dem Regel- und Steuergerät 3 verbunden. Danach wird über den Netzschalter das Regel- und Steuergerät 3 eingeschaltet, das wahlweise mit einer Wechsel-/Gleichspannung zwischen 12 Volt und 240 Volt speisbar ist. Mit dem wahlweisen Drücken des ersten Schalters 46 oder des zweiten Schalters 47 wird eine Temperatur vorgewählt, mit der die Heizauflage 2 beheizt werden soll. Beispielsweise ist dem ersten Schalter 46 eine Temperatur von 34°C zugeordnet und dem zweiten Schalter 47 eine Temperatur von 38°C. Wird nun z.B. der erste Schalter 46 gedrückt, so leuchten in den Leuchtfeldern 40, 41, 42, 43 Leuchtdioden 48, die anzeigen, daß bei störungsfreiem Betrieb die Heizsegmente 12a, 12b, 12c, 12d beheizt werden. Ist nun die Solltemperatur von T = 34°C erreicht, so leuchten in den Leuchtfeldern 40, 41, 42, 42 Leuchtdioden 49 auf, die anzeigen, daß die vorgegebene Heiztemperatur in den einzelnen Heizsegmenten 12a, 12b, 12c, 12d erreicht ist und das Heizungssystem schaltet selbsttätig ab. Fällt die Solltemperatur von T = 34°C ab, so schaltet sich das Heizungssystem wieder ein, so daß die Solltemperatur in den Heizsegmenten immer gegeben ist.

Die in der Heizauflage 2 installierten Temperaturfühler 19 überwachen nun die Temperatur in den einzelnen Heizsegmenten 12a, 12b, 12c, 12d. In jedem Heizsegment wird die Temperatur an zwei Stellen der Heizauflage 2, 30 gemessen und als stetig anfallender Istwert an das Regel- und Steuergerät 3 weitergeleitet. Das Regel- und Steuergerät 3 orientiert sich an dem im Heizsegment höher gemessenen Temperaturwert und regelt danach die Heizleistung der Heizauflage 2,30.

Die elektrische Schaltung des Regel- und Steuergeräts 3 entspricht der in der DE-PS 34 34 772 beschriebenen Schaltung. Sie gilt hiermit für die hier vorliegende Erfindung als erfindungswesentlich offenbart und ist insofern erweitert, als daß Mittel vorgesehen sind, die, bevor eine Regelung eines Heizsegmentes 12a, 12b, 12 c, 12d erfolgt, die zu einem Zeitpunkt gemessenen Temperaturistwerte eines jeden Heizsegmentes 12a, 12b, 12c, 12d miteinander vergleichen. Für die Regelung wird von zwei Temperaturwerten immer der höhere Temperaturwert berücksichtigt.

Wird nun alternativ der zweite Schalter 47 gedrückt, dem beispielsweise eine Temperatur von T = 38°C zugeordnet ist, so wird die Heizauflage 2 solang beheizt, bis die Temperaturfühler 19 eine Temperatur von T = 38°C in der Heizauflage 2 messen. Wird als Istwert von den Temperaturfühlern 19 eine Temperatur von 38°C an das Regel- und Steuergerät 3 übermittelt, so schaltet das Regel- und Steuergerät 3 die Beheizung des Heizpolsters 2 selbsttätig ab und Leuchtdioden 50 leuchten in den Leuchtfeldern 40, 41, 42, 43. Das Regel- und Steuergerät 3 überwacht die Temperatur in den Heizfeldern und hält die Temperatur konstant, d.h. die Beheizung schaltet sich sowohl selbsttätig ein wie auch aus. Wird die Temperatur von T = 38°C in einem der Heizsegmente 12a, 12b, 12c, 12d überschritten und überschreitet die Temperatur einen Grenzwert von beispielsweise T = 41°C, so leuchtet in dem betreffenden Leuchtfeld 40 bis 43 eine Leuchtdiode 51 auf, an die ein akustischer Dauerton gekoppelt ist. Wird der Temperaturgrenzwert von T = 41°C in einem Heizsegment 12a, 12b, 12c, 12d noch weiter überschritten, so leuchtet beispielsweise bei einer in den Heizsegmenten gemessenen Temperatur von T = 45°C eine weitere Leuchtdiode 52 auf, die blinkend und gekoppelt mit einem akustisch unterbrochenen Ton signalisiert, daß die zulässige daß die zulässige Heiztemperatur in der Heizauflage 2 in unzulässiger Weise überschritten worden ist.

Eine weitere Sicherheitsabschaltung ist dann vorgesehen, wenn die Heizauflage 2, 30 länger als z.B. 15 min beheizt wird. Danach schaltet das Regel- und Steuergerät 3 die Beheizung der Heizauflage 2 selbsttätig ab.

Ebenfalls ist das Regel- und Steuergerät 3 dafür geeignet, daß es Widerstandsdifferenzen in den einzelnen Heizsegmenten 12a, 12b, 12c, 12d erkennen kann. Einzelne Heizsegmente 12a, 12b, 12c, 12d oder das gesamte Heizsystem werden dann abgeschaltet, wenn elektrische Leiter 18 fehlerhaft oder unterbrochen sind oder zwischen den elektrischen Leitern 18 eine ungewollte Überbrückung stattfindet.

Die Heizleistung beträgt beispielsweise pro Heizsegment 30 Watt bei einer Spannung von U = 24 Volt.

Die Heizauflage 2, 30 wird in verschiedenen Größen gefertigt, so daß sie für normale OP-Tischplatten wie auch für Kinder-OP-Tischplatten geeignet ist. Ferner kann die Heizauflage 2, 30 als beheizbare Unterlage in allen klinischen Bereichen eingesetzt werden.

## Patentansprüche

1. Wärmesystem für einen Operationstisch mit einer mehrschichtig aufgebauten Heizauflage (2), die mindestens ein elektrisch beheizbares, für Röntgenstrahlen durchlässiges Heizsegment (12a, 12b, 12c, 12d) aufweist, das an ein Regel- und Steuergerät (3) angeschlossen ist, das eine Regelung und Steuerung eines für eine einstellbare Temperatur erforderlichen Heizstromes vermittelt, und die aus unterschiedlichen Schichten gebildet ist, die untereinander einen innigen Materialverbund bilden, dadurch gekennzeichnet, daß die Heizauflage (2) einen Heizleiter (16) aufweist, der einen mit einem bestimmten elektrischen Widerstand behafteten Leiter (18) umfaßt, der auf einen Träger (17) schraubenförmig aufgewickelt ist, der aus einem elektrisch nicht leitenden Material besteht, das hinsichtlich seiner mechanischen Eigenschaften in einem Temperaturbereich von T = 10 Grad Celsius bis T = 200 Grad Celsius gummielastisch ist, daß der Heizleiter (16) in eine erste Schicht (13) eingebettet ist, die eine Dicke aufweist, die dem Außendurchmesser des Heizleiters (16) entspricht, oder bevorzugt 10 bis 20% dicker ist als der Außendurchmesser des Heizleiters (16), daß die erste Schicht (13) von einer zweiten Schicht (14) aus Silikonkautschuk umgeben ist, daß die erste und zweite Schicht (13, 14) weitgehend dieselben Materialeigenschaften aufweisen, und daß die elastischen Ausdehnungen der ersten und zweiten Schicht (13, 14) durch eine zwischen der ersten und zweiten Schicht (13, 14) angeordnete Gewebeschicht (15) begrenzt ist, die mit den angrenzenden Schichten einen Materialverbund bildet, wobei die Gesamtdicke der Heizauflage (2) im Heizsegmentbereich ca. 5 mm und im Randbereich ca. 8 mm beträgt.

2. Wärmesystem nach Anspruch 1, dadurch gekennzeichnet,
daß die Heizauflage (2) einen Heizleiter (16) aufweist, der in der biegetechnisch neutralen Mittelebene der Heizauflage (2) verlegt ist.

3. Wärmesystem nach Anspruch 2, dadurch gekennzeichnet, daß sich der Heizleiter (16) aus einem Träger (17), bevorzugt aus Siliconkautschuk, und aus einem im Querschnitt kreisförmigen Aluminiumdraht als Leiter (18) zusammensetzt.

4. Wärmesystem nach Anspruch 1, dadurch gekennzeichnet,
daß die erste Schicht (13) eine elektrisch nicht leitende Siliconkautschukschicht ist, die eine gute Wärmeleitfähigkeit aufweist und im Materialverbund ein dem Träger (17) ähnliches gummielastisches Verhalten aufweist.

5. Wärmesystem nach Anspruch 1, dadurch gekennzeichnet,
daß die zweite Schicht (14) bevorzugt eine nicht elektrisch leitende Siliconkautschukschicht ist, die im Randbereich verstärkt ist und die über die Oberfläche mit einer weiteren dritten Schicht (25) im Materialverbund steht, die elektrische Ladungen ableiten kann.

6. Wärmesystem nach einem der Ansprüche 1 bis 5, dadurch gekennzeichnet,
daß in der Heizauflage (2) zwischen einem mäanderförmig geführten Heizleiter (16) Temperaturfühler (19), bevorzugt zwei Halbleiterwiderstände mit großen negativen Temperaturkoeffizienten je Heizsegment (12a, 12b, 12c, 12d), angeordnet sind.

7. Wärmesystem nach Anspruch 6, dadurch gekennzeichnet,
daß die Temperaturfühler (19) mit röntgenstrahlendurchlässigen elektrischen Leitern verdrahtet sind.

8. Wärmesystem nach einem dar Ansprüche 1 bis 7, dadurch gekennzeichnet,
daß die elektrischen Leiter der Heizleiter (16) der Heizsegmente (12a, 12b,12c, 12d) in einer Parallelschaltung und die elektrischen Leiter der Temperaturfühler (19) paarweise zusammengefaßt sind, und daß ein elektrischer Leiter für die Ableitung elektrostatischer Aufladungen vorgesehen ist, der mit einer dritten Schicht (25) verbunden ist.

9. Wärmesystem nach Anspruch 8, dadurch gekennzeichnet,
daß die elektrischen Leiter als Leiterbündel am Austritt aus der ersten Schicht (13) einen daran anvulkanisierten Knickschutz aufweisen, der bevorzugt mit einer Knickkerbe (11) versehen ist.

10. Wärmesystem nach Anspruch 9, dadurch gekennzeichnet,
daß das Leiterbündel außerhalb der ersten Schicht (13) mit einer im Bereich von T = 10° Celsius bis T = 200° Celsius temperaturbeständigen und biegsamen Ummantelung umgeben ist und daß das Leiterbündel in einen Stecker (21) mündet, der wahlweise mit einer Kappe (22) dicht verschlossen werden kann.

11. Wärmesystem nach einem der Ansprüche 1 bis 10, dadurch gekennzeichnet,
daß auf der Unterseite der Heizauflage (2; 30) im Randbereich Verschlußbänder, bevorzugt Klettenbänder (23) und/oder Druckknopfbänder (24), vorgesehen sind.

12. Wärmesystem nach einem der Ansprüche 1 bis 11, dadurch gekennzeichnet,
daß die Heizauflage (2) als Heizauflage (30) mit einem Schlitz (31), der sich von einer Querseite der Heizauflage (30) ausgehend teilweise in Längsrichtung erstreckt, ausgebildet ist, an die verschiedene Segmente (33 bis 36) anheftbar und/oder anknüpfbar sind.

13. Wärmesystem nach einem der Ansprüche 1 bis 12, dadurch gekennzeichnet, daß das Regel- und Steuergerät (3) mehrere Eingänge für Leitungen der Heizauflage (2; 30) und der Segmente (33 bis 36) aufweist.

14. Wärmesystem nach einem der Ansprüche 1 bis 13, dadurch gekennzeichnet, daß die Heizauflage (2; 30) und/oder die Segmente (33 bis 36) mit einer Isolationsmatte (4) abdeckbar sind.

15. Wärmesystem nach einem der Ansprüche 1 bis 14, dadurch gekennzeichnet,
daß das Regel- und Steuergerät (3) ein Display (8) aufweist, an dem ein erster und zweiter Schalter (46, 47) für die Temperaturvorwahl der Heizauflage (2; 30) und der Segmente (33 bis 36) und Leuchtfelder (40 bis 45) vorgesehen sind, die den Betrieb, den Betriebszustand und den Störfall der einzelnen Heizsegmente (12a, 12b, 12c, 12d) anzeigen.

## Claims

1. Heating system for an operating table with a heating pad (2), which consists of several layers and comprises at least one heating segment (12a, 12b, 12c, 12d) which can be heated electrically and is permeable to X-rays and is connected to a regulating and controlling device (3), which provides regulation and control of a heating current, which is necessary for a settable temperature, the heating pad (2), being formed of different layers, which form a tight material bond with one another, characterised in that the heating pad (2), comprises of a heat conductor (16), which comprises of a conductor (18), which has a specific electrical resistance and is wound in a spiral manner on a carrier (17), which consists of an electrically non-conductive material, which, with respect to its mechanical properties, is rubber-elastic in a temperature range from T = 10 degrees centigrade to T = 200 degrees centigrade, in that the heat conductor (16) is embedded in a first layer (13), that has a thickness, which corresponds to the external diameter of the heat conductor (16), or is preferably 10 to 20% thicker than the external diameter of the heat conductor (16), in that the first layer (13) is surrounded by a second layer (14) of silicon caoutchouc, in that the first and second layers (13, 14) comprise of essentially the same material properties, and in that the elastic expansions of the first and second layers (13, 14) are limited by a fabric layer (15), which is arranged between the first and second layers (13, 14) and forms a material bond with the adjoining layers, wherein the total thickness of the heating pad (2) in the heating segment area is approximately 5 mm and in the area of the edges approximately 8 mm.

2. Heating system according to claim 1, characterised in that the heating pad (2), comprises of a heat conductor (16), which is positioned in the centre plane of the heating pad (2), which is neutral under bending aspects.

3. Heating system according to claim 2, characterised in that the heat conductor (16) is formed by a carrier (17), preferably of silicon caoutchouc and by an aluminium wire having a circular cross-section and serving as conductor (18).

4. Heating system according to claim 1, characterised in that the first layer (13) is an electrically non-conductive silicon caoutchouc layer, that has a good thermal conductivity and in a material-bonded state has a rubber-elastic behaviour similar to that of the carrier (17).

5. Heating system according to claim 1, characterised in that the second layer (14) is preferably an electrically non-conductive silicon caoutchouc layer, which is reinforced in the area of the edges and the surface of which forms a composite structure with an additional third layer (25), which can discharge electrical charges.

6. Heating system according to any one of claims 1 to 5, characterised in that temperature sensors (19), preferably two semi-conductor resistors with big negative temperature coefficients for each heating segment (12a, 12b, 12c, 12d) are arranged in the heating pad (2), between a heat conductor (16), which is guided in a meandering manner.

7. Heating system according to claim 6, characterised in that the temperature sensors (19), are wired with electrical conductors which are permeable to X-rays.

8. Heating system according to any one of claims 1 to 7, characterised in that the electrical conductors of the heat conductors (16) of the heating segments (12a, 12b, 12c, 12d) are assembled in a parallel circuit and the electrical conductors of the temperature sensors (19) are assembled in pairs and that an electrical conductor, which is connected to a third layer (25), is provided for the discharge of electrostatic charges.

9. Heating system according to claim 8, characterised in that the electrical conductors, as conductor bunch, comprise an anti-kink at the outlet from the first layer (13), which is vulcanized thereto and is preferably provided with a kink notch (11).

10. Heating system according to claim 9, characterised in that the conductor bunch is surrounded outside the first layer (13) by a coating, which is temperature-resistant and flexible within a temperature range of T = 10° centigrade to T = 200° centigrade and that the conductor bunch terminates in a plug (21), which can optionally be closed tightly by means of a cap (22).

11. Heating system according to any one of claims 1 to 10, characterised in that fastening bands, preferably velcro bands (23) and/or press-stud bands (24), are provided in the edge area of the lower side of the heating pad (2; 30).

12. Heating system according to any one of claims 1 to 11, characterised in that the heating pad (2), is constructed as a heating pad (30), with a slit (31), which extends partly from a transverse side of the heating pad (30) in the longitudinal direction, wherein the various segments (33 to 36) are attachable and/or fastenable to the heating pad (2).

13. Heating system according to any one of claims 1 to 12, characterised in that the regulating and controlling device (3) comprises several inlets for the lines of the heating pad (2; 30) and of the segments (33 to 36).

14. Heating system according to any one of claims 1 to 13, characterised in that the heating pad (2; 30) and/or the segments (33 to 36) can be covered by an insulating mat (4).

15. Heating system according to any one of claims 1 to 14, characterised in that the regulating and controlling device (3) comprises a display (8), upon which are provided a first and second switch (46, 47) for preselecting the temperature of the heating pad (2; 30) and of the segments (33 to 36) and luminous fields (40 to 45), which show the operation, operating condition and abnormal occurrence of the individual heating segments (12a, 12b, 12c, 12d).

## Revendications

1. Système de chauffage d'une table d'opération comportant un revêtement chauffant (2), constitué de plusieurs couches, qui comporte au moins un segment chauffant (12a, 12b, 12c, 12d) chauffable électriquement, perméable aux rayons X, qui est connecté à un appareil de régulation et de commande (3) qui opère la régulation et la commande d'un courant de chauffage nécessaire à une température réglable et qui est constitué de différentes couches liées entre elles de façon intime en un matériau composite, caractérisé en ce que le revêtement chauffant (2) comporte un conducteur chauffant (16) qui comprend un conducteur (18) présentant une résistance électrique déterminée, enroulé en hélice sur un support (17) qui est réalisé dans un matériau électriquement non conducteur présentant l'élasticité du caoutchouc, en ce qui concerne ses propriétés mécaniques, dans une plage de températures comprises entre T = 10 degrés Celsius et T = 200 degrés Celsius, en ce que le conducteur chauffant (16) est noyé dans une première couche (13) qui présente une épaisseur correspondant au diamètre extérieur du conducteur chauffant (16), ou de préférence une épaisseur de 10 à 20% supérieure au diamètre extérieur du conducteur chauffant 16, en ce que la première couche (13) est entourée par une deuxième couche (14) en caoutchouc silicone, en ce que la première et la deuxième couches(13, 14) présentent pratiquement les mêmes propriétés de matériau et en ce que l'allongement élastique de la première et de la deuxième couches (13, 14) est limité par une couche de tissu (15), insérée entre la première et la deuxième couches(13, 14), qui forme avec les couches adjacentes un matériau composite, l'épaisseur totale du revêtement chauffant (2) étant égale à 5 mm environ dans la zone du segment chauffant et à 8 mm environ dans la zone de bordure.

2. Système de chauffage selon la revendication 1, caractérisé en ce que le revêtement chauffant (2) comporte un conducteur chauffant (16) qui est posé dans le plan médian, neutre en ce qui concerne les propriétés de flexion, du revêtement chauffant (2).

3. Système de chauffage selon la revendication 2, caractérisé en ce que le conducteur chauffant (16) se compose d'un support (17), de préférence en caoutchouc silicone et d'un fil d'aluminium, de section transversale circulaire, servant de conducteur (18).

4. Système de chauffage selon la revendication 1, caractérisé en ce que la première couche (13) est une couche de caoutchouc silicone, électriquement non conductrice, qui présente une bonne conductibilité thermique et qui, dans le matériau composite, a un comportement présentant l'élasticité du caoutchouc, comme le support (17).

5. Système de chauffage selon la revendication 1, caractérisé en ce que la deuxième couche (14) est de préférence une couche de caoutchouc silicone électriquement non conductrice qui est renforcée dans la zone de bordure et qui est liée, en font un matériau composite,avec une troisième couche (25) qui peut dissiper les charges électriques.

6. Système de chauffage selon l'une des revendications 1 à 5, caractérisé en ce qu'il est prévu dans la couche chauffante (2), entre un conducteur chauffant (16) formant des méandres, des sondes de température (19), de préférence deux résistances à semi-conducteurs présentant des coefficients de température négatifs importants par segment chauffant (12a, 12b, 12c, 12d).

7. Système de chauffage selon la revendication 6, caractérisé en ce que les sondes de température (19) sont câblées avec des conducteurs électriques laissant passer les rayons X.

8. Système de chauffage selon l'une des revendications 1 à 7, caractérisé en ce que les conducteurs électriques des conducteurs chauffants (16) des segments chauffants (12a, 12b, 12c, 12d) sont réunis en un montage parallèle et les conducteurs électriques des sondes de température (19) sont réunis deux par deux et en ce qu'il est prévu un conducteur électrique, relié à une troisième couche (25), pour dissiper les charges électrostatiques.

9. Système de chauffage selon la revendication 8, caractérisé en ce que les conducteurs électriques réunis en faisceau de câbles comportent, à la sortie de la première couche (13), une protection contre le pliage, vulcanisée sur cette couche, qui est pourvue de préférence d'une entaille de pliage (11).

10. Système de chauffage selon la revendication 9, caractérisé en ce que le faisceau de câbles est entouré, à l'extérieur de la première couche (13), d'un gainage flexible et résistant aux températures comprises entre T = 10° Celsius et T = 200° Celsius et en ce que le faisceau de câbles débouche dans un connecteur (21) qui, en option, peut être hermétiquement fermé par un capuchon (22).

11. Système de chauffage selon l'une des revendications 1 à 10, caractérisé en ce que sur la face inférieure du revêtement chauffant (2 ; 30) il est prévu, dans la zone de bordure, des bandes de fermeture, de préférence des bandes à crochets (23) et/ou des bandes à boutons-pression (24).

12. Système de chauffage selon l'une des revendications 1 à 11, caractérisé en ce que le revêtement chauffant (2) est un revêtement chauffant (30) présentant une fente (31) qui s'étend en partie dans la direction longitudinale, à partir d'un côté transversal du revêtement chauffant (30), sur lequel peuvent être accrochés et/ou boutonnés différents segments (33 à 36).

13. Système de chauffage selon l'une des revendications 1 à 12, caractérisé en ce que l'appareil de régulation et de commande (3) comporte plusieurs entrées pour des lignes du revêtement chauffant (2 ; 30) et des segments (33 à 36).

14. Système de chauffage selon l'une des revendications 1 à 13, caractérisé en ce que le revêtement chauffant (2 ; 30) et/ou les segments (33 à 36) peuvent être recouverts d'un matelas isolant (4).

15. Système de chauffage selon l'une des revendications 1 à 14, caractérisé en ce que l'appareil de régulation et de commande (3) comporte un tableau d'affichage (8) sur lequel il est prévu un premier et un deuxième interrupteurs (46, 47) pour la sélection de la température du revêtement chauffant (2 ; 30) et des segments (33 à 36) ainsi que des zones lumineuses (40 à 45) qui indiquent la mise en service, le bon fonctionnement et la défaillance des différents segments chauffants (12a, 12b, 12c, 12d).
